# EUROPEAN PATENT APPLICATION

(11) **EP 4 491 606 A1**
(43) Date of publication of application: **15.01.2025**
(21) Application number: 23860778.2
(22) Date of filing: 24.08.2023
(51) Int. Cl.: C07C 51/43, C07C 57/04

(54) **METHOD FOR PREPARATION OF HIGH PURITY (METH)ACRYLIC ACID**

(30) Priority: 30.08.2022 KR 20220109412; 16.08.2023 KR 20230106990
(71) Applicant: LG Chem, Ltd., Yeongdeungpo-gu Seoul 07336 (KR)
(72) Inventor: YOO, Sung Jin, Daejeon 34122 (KR); JANG, Kyung Soo, Daejeon 34122 (KR); LEE, Sung Kyu, Daejeon 34122 (KR)
(74) Representative: Goddar, Heinz J.
(86) International application number: PCT/KR2023/012550
(87) International publication number: WO 2024/049101

(57) **Abstract**

Provided is a method for preparation of (meth)acrylic acid including: bringing a mixed gas including (meth)acrylic acid into contact with an absorption solvent in an absorption column; supplying a first (meth)acrylic acid solution discharged from a lower portion of the absorption column to a high-boiling point by-product separation column and supplying an upper discharge stream of the high-boiling point by-product separation column to a crystallizer; supplying a second (meth)acrylic acid solution discharged from a side portion of the absorption column to the crystallizer; and obtaining (meth)acrylic acid crystallized in the crystallizer and circulating a mother liquor recovered in the crystallizer to the absorption column.

## Description

### [Technical Field]

### CROSS-REFERENCE TO RELATED APPLICATIONS

The present application claims the benefit of priorities to Korean Patent Application No. 10-2022-0109412, filed on August 30, 2022, and Korean Patent Application No. 10-2023-0106990, filed on August 16, 2023, the entire contents of which are incorporated herein as a part of the specification.

### Technical Field

The present invention relates to a method for preparation of high-purity (meth)acrylic acid.

### [Background Art]

(Meth)acrylic acid is generally prepared by a method of subjecting a compound such as propane, propylene, and (meth)acrolein to a gas phase oxidation reaction in the presence of a catalyst. For example, propane, propylene, and the like are converted through (meth)acrolein into (meth)acrylic acid by a gas phase oxidation reaction in the presence of an appropriate catalyst in a reactor, and a mixed gas including (meth)acrylic acid, unreacted propane or propylene, (meth)acrolein, inert gas, carbon dioxide, water vapor, and various organic by-product by the reaction (such as acetic acid, low-boiling point by-products, and high-boiling point by-products) is obtained in a latter stage of the reactor.

The (meth)acrylic acid-containing mixed gas is brought into contact with an absorption solvent such as water in an absorption column to be recovered as a (meth)acrylic acid solution. Further, as a subsequent process for recovering the (meth)acrylic acid included in the (meth)acrylic acid solution, it is common to involve processes such as extraction, distillation, and purification. In order to improve recovery efficiency of the (meth)acrylic acid, various methods of adjusting process conditions, process order, or the like have been suggested.

However, since the absorption solvent such as water used in the absorption column has a high specific heat, significantly high energy usage is demanded in separating by-products from the (meth)acrylic acid solution including the absorption solvent through a process such as distillation. Meanwhile, when the subsequent process is simplified and shortened for reducing the energy usage, the energy usage may be decreased, but it is difficult to obtain high-purity (meth)acrylic acid.

Therefore, it is urgent to introduce a technology which may reduce the energy usage in separating (meth)acrylic acid and by-products by distillation, while also obtaining high-purity (meth)acrylic acid from a (meth)acrylic acid solution.

### [Disclosure]

### [Technical Problem]

In order to solve the problems mentioned in the Background Art, an object of the present invention is to provide a method for recovering (meth)acrylic acid, which may further reduce energy usage in a purification process and simplify a subsequent process.

### [Technical Solution]

In one general aspect, a method for preparation of (meth)acrylic acid includes: bringing a mixed gas including (meth)acrylic acid into contact with an absorption solvent in an absorption column; supplying a first (meth)acrylic acid solution discharged from a lower portion of the absorption column to a high-boiling point by-product separation column and supplying an upper discharge stream of the high-boiling point by-product separation column to a crystallizer; supplying a second (meth)acrylic acid solution discharged from a side portion of the absorption column to the crystallizer; and obtaining (meth)acrylic acid crystallized in the crystallizer and circulating a mother liquor recovered in the crystallizer to the absorption column.

### [Advantageous Effects]

Since the method for preparation of (meth)acrylic acid according to the present invention may drastically reduce energy usage, high-purity (meth)acrylic acid may be continuously recovered with better production efficiency as compared with previous recovery methods.

### [Description of Drawings]

FIG. 1 is a process flowchart showing a method for preparation of (meth)acrylic acid according to an exemplary embodiment of the present invention.
FIG. 2 is a process flow chart showing a method for preparation of (meth)acrylic acid according to the comparative example.

### [Best Mode]

The terms and words used in the description and claims of the present invention are not to be construed limitedly as having general or dictionary meanings but are to be construed as having meanings and concepts meeting the technical ideas of the present invention, based on a principle that the inventors are able to appropriately define the concepts of terms in order to describe their own inventions in the best mode.

The term "stream" in the present invention may refer to a fluid flow in a process, or may refer to a fluid itself flowing in a pipe. Specifically, the stream may refer to both a fluid itself flowing in a pipe connecting each device and a fluid flow. In addition, the fluid may refer to a gas or liquid, and a case in which a solid substance is included in the fluid is not excluded.

Hereinafter, the present invention will be described in more detail for better understanding of the present invention.

According to the present invention, a method for preparation of (meth)acrylic acid including: bringing a mixed gas including (meth)acrylic acid into contact with an absorption solvent in an absorption column; supplying a first (meth)acrylic acid solution discharged from a lower portion of the absorption column to a high-boiling point by-product separation column and supplying an upper discharge stream of the high-boiling point by-product separation column to a crystallizer; supplying a second (meth)acrylic acid solution discharged from a side portion of the absorption column to the crystallizer; and obtaining (meth)acrylic acid crystallized in the crystallizer and circulating a mother liquor recovered in the crystallizer to the absorption column, is provided.

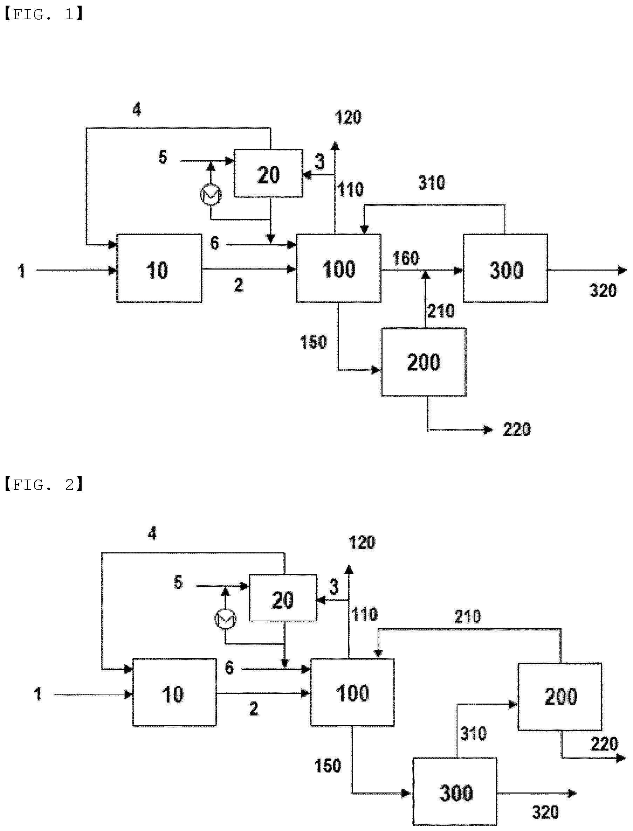

Each process which may be included in the exemplary embodiment of the present invention will be described, with reference to FIG. 1.

First, the method for preparation of (meth)acrylic acid according to an exemplary embodiment of the present invention may include bringing a mixed gas including (meth)acrylic acid into contact with an absorption solvent in an absorption column 100. Herein, the mixed gas including (meth)acrylic acid is a concept which collectively refers to gas phase components discharged from a reactor 10 which produces (meth)acrylic acid by a gas phase oxidation reaction. Specifically, the mixed gas may include (meth)acrylic acid, unreacted raw material compound, (meth)acrolein, inert gas, carbon monoxide, carbon dioxide, absorption solvent steam, various organic by-products (acetic acid, low-boiling point by-products, high-boiling point by-product, etc.), and the like. Herein, a "low-boiling point by-product" (light ends) or a "high-boiling point by-product" (heavies) is a kind of by-product which may be produced in preparation and recovery processes of the target (meth)acrylic acid, and may be a compound having a higher or lower molecular weight than the (meth)acrylic acid.

Specifically, the mixed gas including (meth)acrylic acid may be prepared as follows.

First, a reaction gas including gas containing oxygen and a raw material compound is supplied to a reactor 10 provided with a catalyst through a reaction gas supply line 1, and is subjected to a gas phase oxidation reaction in the presence of a catalyst in the reactor 10 to obtain the mixed gas including (meth)acrylic acid.

Herein, the gas containing oxygen may be air. The raw material compound may be one or more compounds selected from the group consisting of propane, propylene, butane, 1-butylene, t-butylene, and (meth)acrolein, and specifically, the raw material compound may include propylene. Meanwhile, the reaction gas supplied to the reactor 10 may further include a recirculation gas which is recovered from an upper portion of the absorption column 100 and recirculated. Therefore, the mixed gas including (meth)acrylic acid may be a reaction product by the gas phase oxidation reaction of a reactant including air, the raw material compound, and the recirculation gas, in the reactor 10.

The recirculation gas may be derived from the upper portion of the absorption column 100 described later. That is, the mixed gas comes into contact with the absorption solvent in the absorption column 100, and a non-condensable gas which is not dissolved in the absorption solvent may be discharged as an upper discharge stream 110 of the absorption column 100. The non-condensable gas may include impurities such as acetic acid, inert gas, an unreacted raw material compound, a small amount of (meth)acrylic acid which is not dissolved in the absorption solvent, and the like. Meanwhile, a part 3 of the upper discharge stream 110 of the absorption column may be supplied to a cooling tower 20, and the rest 120 may be supplied to a waste gas incinerator and discarded. A content of the (meth)acrylic acid in the upper discharge stream 110 of the absorption column may be 0.03 mol% to 0.5 mol%.

The cooling tower 20 is provided with an absorption solvent supply line 5 in the upper portion, and the absorption solvent may be supplied from the absorption solvent supply line 5 into the cooling tower 20. In the cooling tower 20, the absorption solvent may come into contact with the non-condensable gas included in the part 3 of the upper discharge stream 110 of the absorption column. Components included in the non-condensable gas, for example, acetic acid and (meth)acrylic acid which is not dissolved in the absorption solvent in the absorption column 100 are dissolved in the absorption solvent, and may be discharged as a lower discharge stream of the cooling tower 20.

Thereafter, the lower discharge stream of the cooling tower 20 may be resupplied to the absorption column 100, and the lower discharge stream of the cooling tower 20 may form a mixed stream with a separately supplied absorption solvent stream 6 and be supplied to the absorption column 100.

That is, the supply of the absorption solvent to the absorption column 100 may be performed by one or more supply methods of a supply through an absorption solvent supply line 5 provided in the upper portion of the cooling tower 20 and a direct supply 6 to the absorption column 100, and the direct supply 6 to the absorption column 100 may be a supply to the absorption column 100 after forming a mixed stream with the lower discharge stream of the cooling tower 20. Meanwhile, a supply amount of the absorption solvent supplied to the absorption column 100 may be determined within a range where a content of the (meth) acrylic acid in the lower portion of the absorption column 100 may be maintained at 85 wt% or more.

The absorption solvent supplied to the cooling tower 20 and the absorption solvent supplied to the absorption column 100 may be the same. Specifically, the absorption solvent may include water such as tap water and deionized water, and may include circulation process water introduced from other processes (e.g., water phase recirculated from an extraction process and/or a distillation process). Further, the absorption solvent may include a small amount of organic by-products (e.g., acetic acid) introduced from other processes.

Meanwhile, most of the acetic acid included in the non-condensable gas by a contact with the absorption solvent in the cooling tower 20 is removed by dissolution in the absorption solvent, and gas which is not dissolved in the absorption solvent is discharged through a recirculation gas transfer line 4 provided in the upper portion of the cooling tower 20 as a recirculation gas. The recirculation gas may be supplied to the reactor 10 so that it may be used in the gas phase oxidation reaction for preparation (meth)acrylic acid which proceeds in the reactor 10. The recirculation gas may be mixed with the reaction gas and supplied to the reactor 10, and may be supplied to the reactor 10 through a separate line from the line 1 to which the reaction gas is supplied.

As described above, acetic acid and (meth)acrylic acid in the non-condensable gas supplied from the absorption column 100 are dissolved in the absorption solvent in the cooling tower 20 and may be recirculated to the absorption column 100. Thus, the acetic acid in the system may be induced to be discharged as much as possible to the upper discharge stream 110 of the absorption column to obtain high-purity (meth)acrylic acid and minimize loss of (meth)acrylic acid.

Furthermore, the content of the absorption solvent in the recirculation gas which is circulated from the cooling tower 20 to the reactor 10 may be reduced by lowering a temperature inside the cooling tower 20. That is, by reducing the content of moisture (water) in the recirculation gas when the absorption solvent is water, a moisture content in a stream supplied from the reactor 10 to the absorption column 100 may be lowered, and thus, even the moisture content in the absorption column 100 may be lowered. Various by-products which are inappropriate for being introduced to a crystallizer may be dissolved in the absorption solvent (water) discharged from the reactor, and when the absorption solvent (water) is present in excess in the absorption column 100, it is difficult to obtain a high-concentration (meth)acrylic acid solution, and thus, the content of the absorption solvent (water) in the recirculation gas is lowered, thereby being capable of directly introducing a side discharge stream of the absorption column 100 to the crystallizer, as described later.

When the absorption solvent is water, the moisture content in the recirculation gas may be 1 wt% to 10 wt%, specifically 3 wt% to 5 wt%.

An upper temperature of the cooling tower 20 for this may be 35°C to 55°C, specifically 35°C to 45°C. When the upper temperature of the cooling tower 20 is at least lower than 35°C, an excessive refrigerant is consumed as compared with a reduction effect of the moisture content included in the recirculation gas or a refrigerant at a lower temperature is needed, and thus, there may be no great benefit in terms of efficient energy use. Meanwhile, when the upper temperature of the cooling tower 20 is higher than 55°C, a content of the absorption solvent (moisture) included in the recirculation gas transfer line 4 is excessively increased, and thus, it may be difficult to obtain a high-concentration (meth)acrylic acid solution discharged from the absorption column 100. The temperature of the upper portion of the cooling tower 20 is performed by a heat exchanger provided in the lower portion of the cooling tower 20, and specifically, may be performed by circulating a part of the lower stream of the cooling tower 20 to the heat exchanger and the cooling tower 20. Meanwhile, the upper portion of the cooling tower 20 may be operated under normal pressure operation conditions.

Thereafter, a process for obtaining the (meth)acrylic acid solution by supplying the mixed gas 2 including (meth)acrylic acid to the absorption column 100 through a reactor discharge line and bringing the gas into contact with the absorption solvent in the absorption column 100 may be performed. Specifically, the mixed gas including (meth)acrylic acid produced by a synthesis reaction of (meth)acrylic acid, organic by-products, and absorption solvent steam may be brought into contact with the absorption solvent in the absorption column 100 to obtain the (meth)acrylic acid solution, specifically, first and second (meth)acrylic acid solutions.

Herein, the kind of absorption column 100 may be determined considering contact efficiency of the mixed gas and the absorption solvent and the like, and for example, may be a packed column type absorption column or a multistage tray type absorption column. To the inside of the packed column type absorption column, a filler such as a Rasching ring, a Pall ring, a saddle, a gauze, and a structured packing may be applied.

Further, considering the efficiency of the absorption process, the mixed gas 2 may be supplied to the lower portion of the absorption column 100, and the absorption solvent, specifically the absorption solvent including water may be supplied to the upper portion of the absorption column 100.

Meanwhile, the absorption column 100 may be operated at an internal pressure of 1 to 1.5 bar or 1 to 1.3 bar and at an internal temperature of 50 to 120°C or 50 to 100°C, considering the condensation conditions of (meth)acrylic acid, a moisture content depending on a saturated water vapor pressure, and the like.

Meanwhile, according to an exemplary embodiment of the present invention, through an absorption process performed in the absorption column 100, the first (meth)acrylic acid solution discharged from the lower portion of the absorption column 100 is obtained, and the second (meth)acrylic acid solution discharged from the side portion of the absorption column 100 may be obtained.

The content of the (meth)acrylic acid in the first and second (meth)acrylic acid solutions discharged from the absorption column 100 may be 85 wt% to 99 wt%, specifically 85 wt% to 95 wt%. This is a higher level than the content of the meth)acrylic acid in the (meth)acrylic acid solution discharged from the conventional absorption column. In particular, by setting the content of the (meth)acrylic acid in the second (meth)acrylic acid solution to 85 wt% or more, the second (meth)acrylic acid solution may be directly supplied to the crystallizer 300 without undergoing a separate purification process or separation process for the second (meth)acrylic acid solution, and thus, overall process energy may be reduced, and also, high-purity (meth)acrylic acid may be obtained in the crystallizer 300.

The second (meth)acrylic acid solution having a high (meth)acrylic acid content as such may be achieved by, for example, optimally controlling the operation conditions of the cooling tower 20 and the absorption column 100 depending on the material component in the system and its content to minimize the content of the absorption solvent (moisture) in the absorption column 100. That is, the second (meth)acrylic acid solution having a high (meth)acrylic acid concentration may be implemented by minimizing the absorption solvent component in the recirculation gas which is recirculated from the cooling tower 20 to the reactor 10, minimizing the input amount and used amount of the absorption solvent supplied to the cooling tower 20 and the absorption column 100, and furthermore, for example, setting the number of discharge stages of the second (meth)acrylic acid solution discharged from the absorption column 100.

Specifically, the first (meth)acrylic acid solution may be discharged from the lowest stage of the absorption column 100, and the second (meth)acrylic acid solution may be discharged from the side portion at a height of 80% to 99%, specifically 80% to 90% from top to bottom of the absorption column 100. In particular, when the second (meth)acrylic acid solution is discharged from the side portion at a height of 80% to 99% from top to bottom of the absorption column 100, the absorption solvent (moisture) and the content of the high-boiling point by-product in the discharged second (meth)acrylic acid solution may be minimized. Thus, the second (meth)acrylic acid solution including high-concentration of (meth)acrylic acid may be directly supplied to the crystallizer 300. Specifically, when the second (meth)acrylic acid solution is discharged from a position higher than the position of 80% from top to bottom of the absorption column 100, the content of the absorption solvent discharged through the side portion of the absorption column is increased, and thus, absorption performed at the position lower than the side portion of the absorption column may not be sufficient so that the amount of (meth)acrylic acid lost from the upper portion of the absorption column may be increased. Meanwhile, when the second (meth)acrylic acid solution is discharged from a lower position than the position of 99% from top to bottom of the absorption column 100, the content of the high-boiling point by-product is increased, and thus, it becomes also inappropriate to supply the second (meth)acrylic acid solution to the crystallizer 300.

Meanwhile, the content of the (meth)acrylic acid in the second (meth)acrylic acid solution may be higher than the content of the (meth)acrylic acid in the first (meth)acrylic acid solution. Since relatively heavy high-boiling point by-products are concentrated at the lowest stage portion of the absorption column 100, the content of the (meth)acrylic acid in the second (meth)acrylic acid solution related to the side portion of the absorption column 100 in which almost no high-boiling point by-products are present may be higher than the content of the (meth)acrylic acid in the first (meth)acrylic acid solution.

Meanwhile, the mixed gas may be brought into contact with the absorption solvent in the absorption column 100, a non-condensable gas which is not dissolved in the absorption solvent may be discharged to the upper discharge stream 110 of the absorption column 100, a part 3 of the upper discharge stream 110 of the absorption column may be supplied to the cooling tower 20, and the rest 120 may be supplied to a waste gas incinerator and discarded, as described above.

The first (meth)acrylic acid solution may be supplied to a high-boiling point by-product separation column 200 along a first (meth)acrylic acid solution stream 150, and the second (meth)acrylic acid solution may be supplied to the crystallizer 300 along a second (meth)acrylic acid solution stream 160.

According to an exemplary embodiment of the present invention, the first (meth)acrylic acid solution supplied to the high-boiling point by-product separation column 200 is distilled to be separated into a lower fraction including the high-boiling point by-products and an upper fraction including a high content of (meth)acrylic acid by removing the high-boiling point by-products. The upper fraction of the high-boiling point by-product separation column 200 may be discharged as an upper discharge stream of the high-boiling point by-product separation column and supplied to the crystallizer 300, and the lower fraction of the high-boiling point by-product separation column 200 may be discharged as a lower discharge stream 220 of the high-boiling point by-product separation column and discarded or recycled through a separate purification process. By removing the high-boiling point by-products at a front stage of the crystallizer 300, accumulation of the high-boiling point by-products in the system may be prevented, and a mother liquor discharged from the crystallizer 300 described later may be directly circulated to the absorption column 100.

Since the high-boiling point by-products are removed from the first (meth)acrylic acid solution, the (meth)acrylic acid in the first (meth)acrylic acid solution is more concentrated, and thus, the content of the (meth)acrylic acid in the upper discharge stream of the high-boiling point by-product separation column 200 may be 85 wt% to 99 wt%, 90 wt% to 99 wt%, or specifically 90 wt% to 95 wt%.

Meanwhile, according to an exemplary embodiment of the present invention, the side discharge stream 160 of the absorption column 100 including the second (meth)acrylic acid solution and the upper discharge stream 210 of the high-boiling point by-product separation column 200 may be supplied to the crystallizer 300.

The (meth)acrylic acid included in the (meth)acrylic acid solution supplied to the crystallizer 300 may be recrystallized through a crystallization process and obtained as recrystallized high-purity (meth)acrylic acid. The crystallization process may be performed under common conditions.

The crystallization method for obtaining a product by crystallization in the present invention may be suspension crystallization and layer crystallization without limitation, may be continuous or batchwise, and may be performed in one stage or two stages or more. As a non-limited example, the (meth)acrylic acid is dynamically crystallized to be provided as high-purity (meth)acrylic acid.

Specifically, in order to dynamically crystallize the (meth)acrylic acid before crystallization, the (meth)acrylic acid solution may be first allowed to flow on a tube inner wall in a falling film form. Further, the temperature of the tube may be adjusted to a freezing point of (meth)acrylic acid or lower to form a crystal in the tube inner wall. Then, the temperature of the tube may be raised to near the freezing point of (meth)acrylic acid to sweat about 5 wt% of (meth)acrylic acid. Further, the mother liquor sweated from the tube is removed and the crystal formed in the tube inner wall is recovered, thereby obtaining the high-purity crystallized (meth)acrylic acid. The mother liquor may refer to a solution which is the (meth)acrylic acid solution introduced to the crystallizer 300 from which the crystallized (meth)acrylic acid has been removed. The mother liquor may include acetic acid, an absorption solvent, and a low-boiling point material.

Separation of the mother liquor and the crystallized (meth)acrylic acid may be performed using a solid-liquid separation device, for example, a belt filter, a centrifuge, and the like.

According to an exemplary embodiment of the present invention, the mother liquor may be discharged from the crystallizer and circulated to the absorption column 100 along a mother liquor circulation line 310. The mother liquor may be directly supplied to the absorption column 100 without undergoing a separate purification process or separation process. That is, a ratio of a flow rate when the mother liquor is introduced to the absorption column to a flow rate when the mother liquor recovered in the crystallizer 300 is discharged from the crystallizer may be 0.99 to 1.01, more specifically 1. Meanwhile, by not performing a separate process for the mother liquor, for example, a distillation process, the process is simplified, and the absorption solvent included in the mother liquor, specifically water, does not need to be distilled, and thus, energy costs may be greatly decreased.

Hereinafter, the present invention will be described in more detail by the examples. However, the following examples are provided for illustrating the present invention, and it is apparent to a person skilled in the art that various modifications and alterations may be made without departing from the scope and spirit of the present invention, and the scope of the present invention is not limited thereto.

### Example 1

According to the process flow illustrated in FIG. 1, a preparation process of (meth)acrylic acid was simulated, using an Aspen Plus simulator available from Aspen.

Specifically, a mixed gas 2 having a composition of (meth)acrylic acid (7.0 mol%), water (11.8 mol%), a high-boiling point material (0.09 mol%), and inert gas (80.6 mol%) was obtained by a gas phase oxidation reaction of a reactant including air (oxygen), a raw material compound (propylene), and a recirculation gas 4 in the presence of a catalyst in a reactor 10.

Subsequently, the mixed gas 2 was added to a 22nd stage from the top of an absorption column 100 at a temperature of 164°C. A pressure in the upper portion of the absorption column 100 was 1.1 bar, a temperature in the lower portion of the absorption column 100 was 96.4°C, and an absorption solvent (water) added to the absorption column 100 was supplied through a stream 6 directly supplied to the absorption column and a lower discharge stream of the cooling tower 20. The absorption solvent was added to the upper portion of the absorption column 100 at a flow rate of 6.6 wt% with respect to the flow rate of the mixed gas 2.

Meanwhile, the mother liquor 310 circulated from the crystallizer described later was added to the 15th stage position from the top of the absorption column 100 at a mass flow rate of 1.3 times the flow rate of the absorption solvent introduced to the absorption column 100. A ratio of the mass flow rate when the mother liquor recovered from the crystallizer 300 was introduced to the absorption column 100 to the flow rate when the recovered mother liquor was discharged from the crystallizer 300 was 1.

An upper discharge stream 110 of the absorption column including a non-condensable gas, a first (meth)acrylic acid solution stream 150 including the first (meth)acrylic acid solution, and a second (meth)acrylic acid solution stream 160 including the second (meth)acrylic acid solution were obtained by an absorption process performed in the absorption column 100. A content of the (meth)acrylic acid included in the upper discharge stream 110 of the absorption column was 0.146 mol%.

A part 3 of the upper discharge stream of the absorption column was supplied to a cooling tower 20, and the rest 120 was discarded out of the system. The non-condensable gas included in the part 3 of the upper discharge stream of the absorption column and the absorption solvent supplied from the absorption solvent supply line 5 were brought into contact in the cooling tower 20, and a lower discharge stream of the cooling tower 20 including the absorption solvent and components dissolved in the absorption solvent (acetic acid and (meth)acrylic acid which was not dissolved in the absorption solvent in the absorption column) were obtained and gas (recirculation gas) which was not dissolved in the absorption solvent was supplied to the reactor 10 through a recirculation gas transfer line 4. A part of the lower discharge stream of the cooling tower 20 was supplied to the absorption column 100, and the rest was circulated to the cooling tower 20. At this time, a moisture content of the recirculation gas 4 was 4.4 wt%.

Meanwhile, the first (meth)acrylic acid solution 150 discharged from the bottom of the absorption column 100 included (meth)acrylic acid (85.9 wt%), acetic acid (1.3 wt%), water (4.2 wt%), furfural (3.2 wt%), and maleic acid (5.0 wt%), and the second (meth)acrylic acid solution 160 discharged from the side portion at a height of 86% from the top of the absorption column included (meth)acrylic acid (88.8 wt%), acetic acid (2.3 wt%), water (5.3 wt%), furfural (3.1 wt%), and maleic acid (0.06 wt%). At this time, the second (meth)acrylic acid solution stream 160 was obtained at a flow rate of 7.3 times the mass flow rate of the first (meth)acrylic acid solution stream 150.

Meanwhile, the mass flow rate of the absorption solvent included in the first and second (meth) acrylic acid solutions was increased by 30 wt% with respect to the flow rate of the absorption solvent introduced to the absorption column 100.

The first (meth)acrylic acid solution stream 150 was supplied to the high-boiling point by-product separation column 200, the high-boiling point by-products was separated through the lower discharge stream 220 of the high-boiling point by-product separation column, and a mixed stream of the upper discharge stream 210 of the high-boiling point by-product separation column including (meth)acrylic acid with the second (meth) acrylic acid solution stream 160 was formed, which was then supplied to the crystallizer 300. Here, the content of (meth)acrylic acid in the upper discharge stream 210 of the high-boiling point by-product separation column was 92.8 wt%. Meanwhile, the mixed stream of the upper discharge stream 210 of the high-boiling point by-product separation column and the second (meth)acrylic acid solution stream 160 included furfural (2.8 wt%) and maleic acid (0.06 wt%) as the high-boiling point by-products.

The mixed stream of the upper discharge stream 210 of the high-boiling point by-product separation column and the second (meth)acrylic acid solution stream 160 was crystallized in the crystallizer 300, and (meth)acrylic acid was finally obtained from a (meth)acrylic acid recovery stream 320 including the (meth)acrylic acid and a mother liquor was supplied to the absorption column 100 through a mother liquor circulation line 310. The content of the (meth)acrylic acid included in the (meth)acrylic acid recovery stream 320 was 99.5 wt% or more.

At this time, an energy of 36.4 kcal/kg AA was used in the high-boiling point by-product separation column 200, and 99.5 wt% or more of (meth)acrylic acid was obtained from the crystallizer. Meanwhile, a loss amount of (meth)acrylic acid lost through the upper discharge stream 110 of the absorption column was 0.146 mol%.

### Example 2

In Example 2, the (meth)acrylic acid was prepared by the same process flow as in Example 1, except that the second (meth)acrylic acid solution discharged from the height of 77% from top to bottom of the absorption column 100 was supplied to the crystallizer.

At this time, the second (meth)acrylic acid solution included (meth)acrylic acid (88.8 wt%), acetic acid (2.2 wt%), water (5.4 wt%), furfural (3.2 wt%), and maleic acid (0.04 wt%).

As a result, the content of the (meth)acrylic acid in the (meth)acrylic acid recovery stream 320 was 99.5 wt% or more, thereby obtaining the (meth)acrylic acid. The content of the (meth)acrylic acid included in the upper discharge stream 110 of the absorption column was 0.25 mol%.

As such, when a discharge height of the side discharge stream of the absorption column was controlled to be higher than Example 1, the loss amount of the (meth)acrylic acid discharged as the upper discharge stream of the absorption column was 0.25 mol%, which was confirmed to be increased as compared with the loss amount of the (meth)acrylic acid in Example 1.

### Comparative Example

### Comparative Example 1

According to the process flow illustrated in FIG. 2, a preparation process of (meth)acrylic acid was simulated, using an Aspen Plus simulator available from Aspen.

Specifically, in Comparative Example 1, the (meth)acrylic acid solution was not obtained from the side portion and the bottom of the absorption column 100, respectively, but obtained only from the bottom of the absorption column, and was added to the crystallizer 300 to obtain (meth)acrylic acid 320. The mother liquor 310 after crystallization was added to the high-boiling point by-product separation column 200 to obtain a lower discharge stream 220 of the high-boiling point by-product separation column including the high-boiling point by-products and an upper discharge stream 210 of the high-boiling point by-product separation column including the mother liquor from which the high-boiling point by-products were removed. The upper discharge stream 210 of the high-boiling point by-product separation column was recirculated to the absorption column 100. The (meth)acrylic acid was prepared in the same manner as in Example 1, except the above.

The (meth)acrylic acid solution included (meth)acrylic acid (90.5 wt%), acetic acid (1.8 wt%), water (5.9 wt%), furfural (0.7 wt%), and maleic acid (0.7 wt%). The (meth)acrylic acid solution was added to the crystallizer 300, and it was confirmed that the content of (meth)acrylic acid in the (meth)acrylic acid recovery stream 320 was 99.5 wt% or more.

For the mother liquor after crystallization, energy used for removing the high-boiling point by-products in the high-boiling point by-product separation column 200 was 154.5 kcal/kg AA, and it was confirmed therefrom that about 4 times more energy than energy used in the high-boiling point by-product separation column 200 of Example 1 was consumed. Meanwhile, the content of the (meth)acrylic acid (loss amount of acrylic acid) included in the upper discharge stream 110 of the absorption column was 0.144 mol%.

## Claims

1. A method for preparation of (meth)acrylic acid, the method comprising:
bringing a mixed gas including (meth)acrylic acid into contact with an absorption solvent in an absorption column;
supplying a first (meth)acrylic acid solution discharged from a lower portion of the absorption column to a high-boiling point by-product separation column and supplying an upper discharge stream of the high-boiling point by-product separation column to a crystallizer;
supplying a second (meth)acrylic acid solution discharged from a side portion of the absorption column to the crystallizer; and
obtaining (meth)acrylic acid crystallized in the crystallizer and circulating a mother liquor recovered in the crystallizer to the absorption column.

2. The method for preparation of (meth)acrylic acid of claim 1, wherein a content of the (meth)acrylic acid in the second (meth)acrylic acid solution is higher than a content of the (meth)acrylic acid in the first (meth)acrylic acid solution.

3. The method for preparation of (meth)acrylic acid of claim 1, wherein the content of the (meth)acrylic acid in the first and second (meth)acrylic acid solutions is 85 wt% to 99 wt%.

4. The method for preparation of (meth)acrylic acid of claim 1, wherein a ratio of a flow rate when the recovered mother liquor is introduced to the absorption column to a flow rate when the mother liquor recovered from the crystallizer is discharged from the crystallizer is 0.99 to 1.01.

5. The method for preparation of (meth)acrylic acid of claim 1, wherein a content of the (meth)acrylic acid in an upper discharge stream of the high-boiling point by-product separation column is 90 wt% to 99 wt%.

6. The method for preparation of (meth)acrylic acid of claim 1,
wherein the first (meth)acrylic acid solution is discharged from a lowest stage of the absorption column, and
the second (meth)acrylic acid solution is discharged from a side portion at a height of 80% to 99% from top to bottom of the absorption column.

7. The method for preparation of (meth)acrylic acid of claim 1, wherein a content of the (meth)acrylic acid in the upper discharge stream of the absorption column is 0.03 mol% to 0.5 mol%.

8. The method for preparation of (meth)acrylic acid of claim 1, wherein a flow rate of the absorption solvent included in the first and second (meth)acrylic acid solutions with respect to a flow rate of the absorption solvent introduced to the absorption column is 30 wt% to 50 wt%.

9. The method for preparation of (meth)acrylic acid of claim 1,
wherein the mixed gas including (meth)acrylic acid is a reaction product by a gas phase oxidation reaction of a reactant including air, a raw material compound, and a recirculation gas in the reactor, and
the recirculation gas is that, after a part of the upper discharge stream of the absorption column is supplied to the cooling tower and cooled, discharged as an upper discharge stream of the cooling tower and circulated to the reactor.

10. The method for preparation of (meth)acrylic acid of claim 9,
wherein the absorption solvent is water, and
a moisture content in the recirculation gas is 1 wt% to 10 wt%.
